# EUROPEAN PATENT SPECIFICATION

(11) **EP 2 910 273 B1**
(45) Date of publication and mention of the grant of the patent: **04.05.2016**
(21) Application number: 15154084.6
(22) Date of filing: 06.02.2015
(51) Int. Cl.: A61M 25/00, A61M 25/01, A61M 25/10

(54) **Catheter**
Katheter
Cathéter

(30) Priority: 24.02.2014 JP 2014033196
(43) Date of publication of application: 26.08.2015
(73) Proprietor: ASAHI INTECC CO., LTD., Nagoya-shi, Aichi 463-0024 (JP)
(72) Inventor: Nishigishi, Makoto, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 2 495 006
- US-A- 5 545 134
- US-A1- 2003 208 221
- US-A1- 2004 019 324

## Description

### [Technical Field]

The present invention relates to a catheter that can efficiently transmit a pressing force of an operator to the end of the catheter without being bent when being pressed by the operator in the distal direction of the catheter.

### [Background Art]

Conventionally, catheters are widely used for treating stenoses in blood vessels or alimentary canals. In order to press a catheter to a stenosis, a pressing force applied in the distal direction of the catheter needs to be efficiently transmitted to the end of the catheter.

As a method for such transmission, a known catheter has a reinforcing member (core wire) in the longitudinal direction between an outer tube and an inner tube (For example, see Patent Literature 1). In the catheter of Patent Literature 1, however, only the reinforcing member is inserted in the longitudinal direction. Unfortunately, this leads to insufficient stiffness and thus a pressing force applied by an operator in the distal direction is hardly transmitted to the end of the catheter.

Another known catheter includes a metal tube having high stiffness on the rear end operated by an operator and a reinforcing member joined to the end of the metal tube (For example, see Patent Literature 2). Since the stiff metal tube is provided on the rear end, a pressing force applied by the operator in the distal direction can be transmitted from the metal tube to the end of the catheter through the reinforcing member.

In the catheter of Patent Literature 2, however, the rear end of the reinforcing member is joined to the inner peripheral surface of the metal tube and thus another medical device inserted into the metal tube may be caught at the rear end of the reinforcing member.

If the rear end of the reinforcing member is directly joined to the outer peripheral surface of the metal tube to facilitate the insertion of another medical device into the metal tube, the metal tube improves the stiffness of the rear end and increases the diameter of the outer tube. Unfortunately, this may increase the diameter of the overall catheter so as to prevent the catheter from reaching a site with stenosis on the end of a blood vessel or an alimentary canal. Alternatively, in order not to increase the diameter of the outer tube, the reinforcing member to be inserted into the outer tube needs to be reduced in diameter. Unfortunately, a reduction in the diameter of the reinforcing member may cause insufficient stiffness in the longitudinal direction of the catheter, causing a break on the catheter pressed by an operator in the distal direction.

Other known catheters are all disclosed in US 2004/019324 A1, US 5545134 A, US 2003/208221 A1, and EP 2495006 A1.

### [Citation List]

### [Patent Literature]

[Patent Literature 1] WO 2006/126642
[Patent Literature 2] Japanese Patent Laid-Open No. 2003-164528
[Patent Literature 3] US 2004/019324 A1
[Patent Literature 4] US 5545134 A
[Patent Literature 5] US 2003/208221 A1
[Patent Literature 6] EP 2495006 A1

### [Summary of Invention]

### [Technical Problem]

The present invention has been devised in view of these circumstances. An object of the present invention is to provide a catheter as described in the claims in which a metal tube has a lower first wall and an upper second wall opposed to the lower first wall, a front end of the lower first wall is distally extended longer than that of the upper second wall and is bent toward the upper second wall, thereby increasing the diameter of a reinforcing member by the thickness of the upper second wall of the metal tube without increasing the diameter of the outer tube, so that a pressing force applied by the operator can be efficiently transmitted to the front end of the catheter without causing a break on the catheter.

### [Solution to Problem]

The object is attained by the following solutions:

A first aspect of the present invention includes an outer tube, a metal tube inserted into the outer tube, an inner tube inserted into the metal tube, and a metallic reinforcing member extended in a longitudinal direction between the outer tube and the inner tube, wherein the metal tube has a lower first wall and an upper second wall opposed to the lower first wall, a front end of the lower first wall is distally extended longer than that of the upper second wall and is bent toward the upper second wall, and the reinforcing member has a rear end that is joined to the outer peripheral surface of the bent lower first wall..

A second aspect of the present invention is the catheter according to the first aspect, wherein the rear end of the reinforcing member has a recess, and the front end of the lower first wall is joined to the reinforcing member at the recess.

A third aspect of the present invention is the catheter according to the first or second aspect, further including a balloon attached to the front end of the inner tube, and a liquid supply tube provided along the inner tube so as to supply a liquid into the balloon, wherein the liquid supply tube has a rear end that is connected to a lumen formed between the outer tube and the inner tube.

### [Advantageous Effects of Invention]

In the catheter according to the first aspect of the present invention, a metal tube has a lower first wall and an upper second wall opposed to the lower first wall, a front end of the lower first wall is distally extended longer than that of the upper second wall and is bent toward the upper second wall, and a rear end of a reinforcing member is joined to an outer peripheral surface of the bent lower first wall. The upper second wall of the metal tube is not provided at a connected portion between the lower first wall of the metal tube and the rear end of the reinforcing member. Thus, the reinforcing member increased in diameter by the thickness of the upper second wall of the metal tube can be disposed in the catheter without increasing the diameter of the outer tube. Hence, when the operator presses the catheter in the distal direction, the catheter is unlikely to have insufficient stiffness in the longitudinal direction so as to reduce the occurrence of breaks on the catheter. This can efficiently transmit a pressing force, which is applied by the operator in the distal direction, to the front end of the catheter.

In the catheter according to the second aspect of the present invention, the front end of the lower first wall of the metal tube is joined to the recess on the rear end of the reinforcing member. Thus, the connected portion between the rear end of the reinforcing member and the lower first wall of the metal tube may be the front end of the lower first wall as well as the outer peripheral surface of the lower first wall. This can improve bonding strength between the reinforcing member and the metal tube. The provision of the recess on the rear end of the reinforcing member can further increase the diameter of the reinforcing member in contact with the lower first wall of the metal tube. This can efficiently transmit a pressing force, which is applied by the operator in the distal direction, to the front end of the catheter.

The catheter according to the third aspect of the present invention further includes a balloon attached to the front end of the inner tube, and a liquid supply tube provided along the inner tube so as to supply a liquid into the balloon, wherein the liquid supply tube has a rear end that is connected to a lumen formed between the outer tube and the inner tube. Thus, a liquid can be supplied into the balloon through the lumen formed between the outer tube and the inner tube and the liquid supply tube, inflating the balloon so as to fix the front end of the catheter. This also fixes the front end of the inner tube and improves the operability of another medical device inserted into the inner tube.

In the catheter according to the third aspect of the present invention, the liquid supply tube for supplying a liquid into the balloon is provided on the front end of the catheter. When the catheter is inserted into a greatly curved blood vessel or alimentary canal, the front end of the inner tube is considerably curved. Thus, when the operator inserts another medical device into the inner tube, the medical device may erroneously break the front end of the inner tube. Even in this case, a liquid can be supplied into the balloon through the special liquid supply tube.

### [Brief Description of Drawings]

FIG. 1 is an overall view of a catheter according to a first embodiment.
FIG. 2 (A) is a cross-sectional view taken along line A-A of FIG. 1, FIG. 2 (B) is a cross-sectional view taken along line B-B of FIG. 1, FIG. 2(C) is a cross-sectional view taken along line C-C of FIG. 1, and FIG. 2 (D) is a cross-sectional view taken along line D-D of FIG. 1.
FIG. 3 is an overall view of a catheter according to a second embodiment, a modification of FIG. 1.
FIG. 4 (A) is a cross-sectional view taken along line A-A of FIG. 3, FIG. 4 (B) is a cross-sectional view taken along line B-B of FIG. 3, FIG. 4(C) is a cross-sectional view taken along line C-C of FIG. 3, and FIG. 4 (D) is a cross-sectional view taken along line D-D of FIG. 3.
FIG. 5 is an overall view of a catheter according to a third embodiment, a modification of FIG. 3.
FIG. 6 (A) is a cross-sectional view taken along line A-A of FIG. 5, FIG. 6 (B) is a cross-sectional view taken along line B-B of FIG. 5, FIG. 6(C) is a cross-sectional view taken along line C-C of FIG. 5, and FIG. 6 (D) is a cross-sectional view taken along line D-D of FIG. 5.

### [Description of Embodiments]

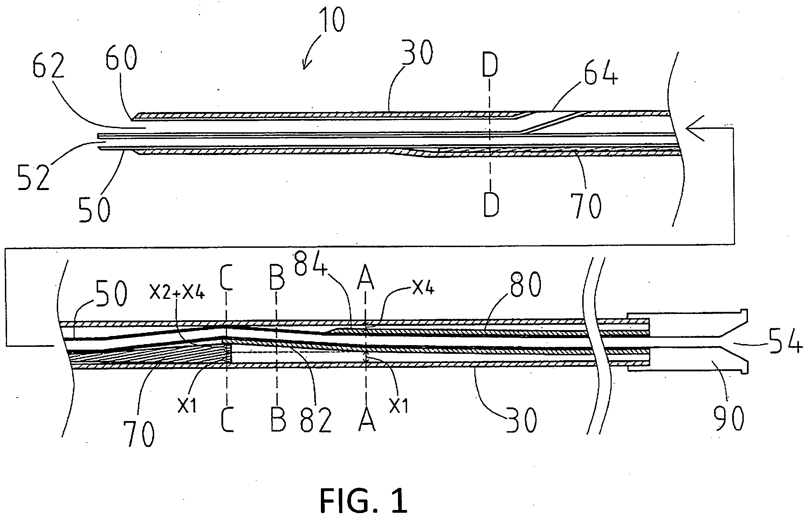

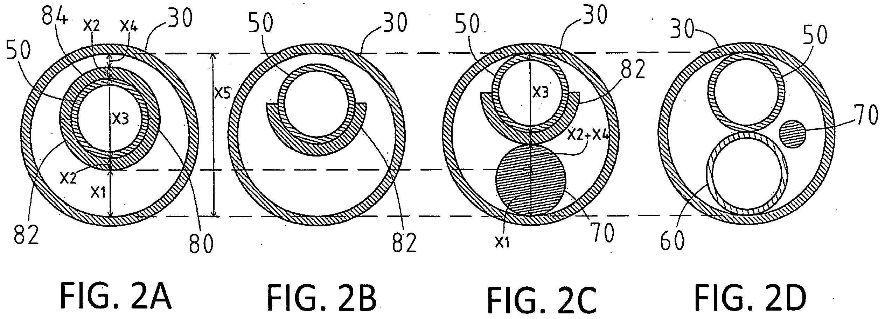

Referring to FIGS. 1 to 2(D), a catheter 10 according to the present embodiment will be described in the following example. In FIG. 1, the left side indicates a front end (distal side) to be inserted into a body while the right side indicates a rear end (proximal side) operated by an operator, e.g., a doctor.

For example, the catheter 10 is used for treating a stenosis formed in a blood vessel or an alimentary canal. As shown in FIG. 1, the catheter 10 mainly includes an outer tube 30, a first inner tube 50, a second inner tube 60, a reinforcing member 70, a metal tube 80, and a connector 90.

In the outer tube 30, the first inner tube 50 is inserted along the length of the catheter 10. Another medical device, e.g., a guide wire or a microcatheter can be inserted into the first inner tube 50. In order to facilitate the insertion of another medical device, a connector 90 is connected to the rear end of the outer tube 30 and the rear end of the first inner tube 50. The front end of the first inner tube 50 has a first front-end opening 52 while a first insertion opening 54 is provided on the rear end of the first inner tube 50 via the connector 90.

In the outer tube 30, the second inner tube 60 is inserted from a midpoint to the front end of the catheter 10 in parallel with the first inner tube 50. As in the first inner tube 50, another medical device, e.g., a guide wire or a microcatheter can be inserted into the second inner tube 60. The front end of the second inner tube 60 has a second front-end opening 62 while the rear end of the second inner tube 60 has a second insertion opening 64.

Since the first inner tube 50 is extended along the length of the catheter 10, the length of the first inner tube 50 may disadvantageously lead to difficulty in replacement of another medical device to be inserted by an operator. However, the insertion of another medical device into the first inner tube 50 may improve the stiffness of the catheter 10, advantageously allowing an operator to easily press the catheter 10 in the distal direction. Moreover, the second inner tube 60 is only extended from a midpoint to the front end of the catheter 10 and thus has a short length, advantageously allowing the operator to easily change another medical device inserted into the second inner tube 60. However, even if another medical device is inserted into the second inner tube 60, only the front end of the catheter 10 increases in stiffness, disadvantageously causing a break on the catheter 10 around the second insertion opening 64 of the second inner tube 60 that rapidly fluctuates in stiffness when the operator presses the catheter 10 in the distal direction. Since the catheter 10 includes the first inner tube 50 and the second inner tube 60, the operator can quickly change another medical device inserted into the second inner tube 60 while another medical device is inserted into the first inner tube 50. Furthermore, the catheter 10 is easily pressed in the distal direction.

A metal tube 80, a so-called hypotube, is inserted into the rear end of the outer tube 30. The metal tube 80 is made of stainless steel or superelastic alloys such as a Ni-Ti alloy, providing stiffness on the rear end of the catheter 10. The rear end of the metal tube 80 is connected to the connector 90. The metal tube 80 has a lower first wall 82 and an upper second wall 84 opposed to the lower first wall 82. A front end of the lower first wall 82 is distally extended longer than that of an upper second wall 84. The front end of the lower first wall 82 is bent to the upper second wall 84. The first inner tube 50 is inserted into the metal tube 80.

FIG. 2 (A) is a cross-sectional view taken along line A-A of FIG. 1. FIG. 2 (B) is a cross-sectional view taken along line B-B of FIG. 1. FIG. 2(C) is a cross-sectional view taken along line C-C of FIG. 1. In FIG. 2(A), the metal tube 80 has a circular shape composed of the lower first wall 82 and the upper second wall 84. In FIGS. 2(B) and 2(C), the metal tube 80 has a semicircular shape only composed of the lower first wall 82 because the upper second wall 84 is cut off from the front end of the metal tube 80.

The catheter 10 further includes the metallic reinforcing member 70 that is longitudinally extended between the outer tube 30 and the first inner tube 50 (See FIG. 1). The reinforcing member 70 is a tapered metallic wire rod that is circular in cross section and is reduced in diameter toward the front end. The material of the reinforcing member 70 is not particularly limited. For example, stainless steel or superelastic alloys such as a Ni-Ti alloy can be used.

The reinforcing member 70 is longitudinally extended along the first inner tube 50. The front end of the reinforcing member 70 is extended longer than the second insertion opening 64 of the second inner tube 60 toward the front end of the catheter 10 (See FIGS. 1 and 2(D)). The rear end of the reinforcing member 70 is joined to the outer peripheral surface of the lower first wall 82 that is bent toward the upper second wall 84 (See FIGS. 1 and 2(C)).

In FIG. 2(A), X1 is a lower clearance between the outer peripheral surface of the metal tube 80 (lower first wall 82) and the inner peripheral surface of the outer tube 30, X2 is the thickness of the metal tube 80 (in other words, the thickness of the lower first wall 82 and the thickness of the upper second wall 84), X3 is the outside diameter of the first inner tube 50, X4 is an upper clearance between the outer peripheral surface of the metal tube 80 (upper second wall 84) and the inner peripheral surface of the outer tube 30, and X5 is the inside diameter of the outer tube 30. The relationship of X5 = X1 + X2 + X3 + X2 + X4 is established. The total of the clearances between the outer peripheral surface of the metal tube 80 and the inner peripheral surface of the outer tube 30 is expressed as X1 (the clearance on the lower first wall 82) + X4 (the clearance on the upper second wall 84). Thus, when the rear end of the reinforcing member 70 is joined to the outer peripheral surface of the metal tube 80, the maximum diameter of the reinforcing member 70 is X1 + X4.

As shown in FIG. 2 (C), in the catheter 10, the upper second wall 84 is cut off and the lower first wall 82 is bent toward the upper second wall 84. Thus, although the inside diameter X5 of the outer tube 30 is kept constant in the longitudinal direction (in other words, without increasing the diameter of the outer tube 30), the diameter of the reinforcing member 70 is X5 - X3 (the outside diameter of the first inner tube 50) - X2 (the thickness of the lower first wall 82) = X1 + X2 + X4. Thus, the reinforcing member 70 increased in diameter by the thickness X2 of the upper second wall 84 of the metal tube 80 can be disposed in the catheter 10 without increasing the diameter of the outer tube 30. Hence, when an operator presses the catheter 10 in the distal direction, the catheters 10 is unlikely to have insufficient stiffness in the longitudinal direction so as to reduce the occurrence of breaks on the catheter 10. This can efficiently transmit a pressing force, which is applied by the operator in the distal direction, to the front end of the catheter 10.

In FIGS. 1 and 2 (B), the upper second wall 84 is completely cut off. The present invention is not limited to this configuration. For example, the upper second wall 84 having the thickness X2 in the cross-sectional view taken along line A-A of FIG. 1 may be gradually reduced in thickness toward the front end such that the thickness of the upper second wall 84 is smaller than X2 in the cross-sectional view taken along line B-B of FIG. 1 and the upper second wall 84 is not provided in the cross-sectional view taken along line C-C of FIG. 1. This configuration can gradually change stiffness from the metal tube 80 to the rear end of the reinforcing member 70.

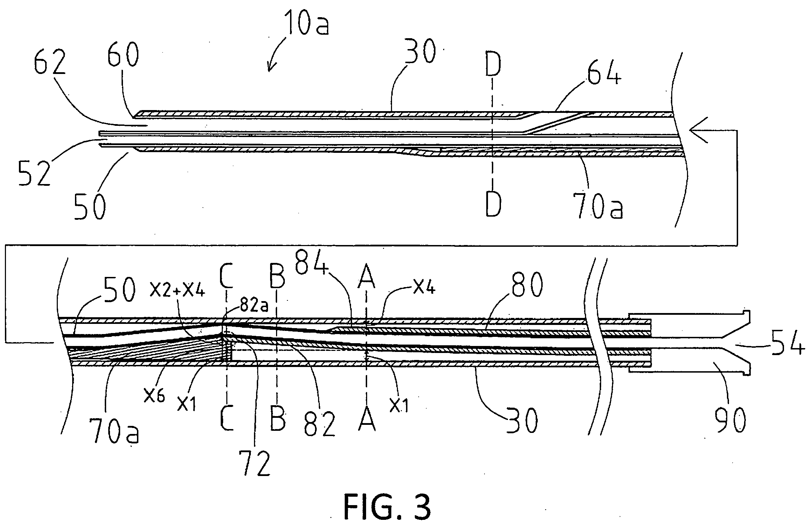

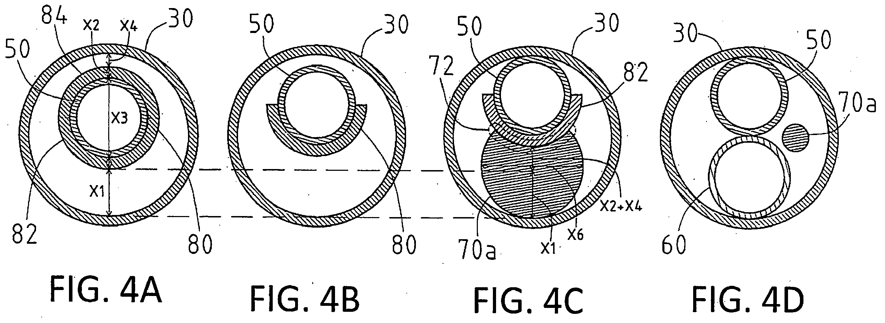

Referring to FIGS. 3 to 4(D), a catheter 10a according to a second embodiment will be described below. In FIG. 3, as in FIG. 1, the left side indicates a front end (distal side) to be inserted into a body while the right side indicates a rear end (proximal side) operated by an operator, e.g., a doctor.

Only differences from the catheter 10 of FIGS. 1 to 2 (D) will be described below. In the catheter 10a, a recess 72 is formed on the rear end of a reinforcing member 70a while the front end of a lower first wall 82 of a metal tube 80 is joined to the recess 72 of the reinforcing member 70a (See FIGS. 3 and 4 (C)). Like the reinforcing member 70, the reinforcing member 70a is a tapered metallic wire rod that is circular in cross section and is reduced in diameter toward the front end. The reinforcing member 70a has a diameter X6 that is maximized at a contact point with a front end 82a of the lower first wall 82 of the metal tube 80. The diameter X6 is larger than the diameter X1 + X2 + X4 of the reinforcing member 70 (X6 > X1 + X2 + X4). The recess 72 of the reinforcing member 70a is formed so as to fit to the front end 82a of the lower first wall 82. The reinforcing member 70a on the recess 72 has a diameter of X1 + X2 + X4 like the reinforcing member 70 (See FIG. 4(C)).

With this configuration, the catheter 10a has the recess 72 on the rear end of the reinforcing member 70a, further increasing the diameter X6 of the reinforcing member 70a in contact with the front end 82a of the lower first wall 82 of the metal tube 80. Moreover, the rear end of the reinforcing member 70a and the lower first wall 82 of the metal tube 80 are joined to each other on the front end 82a of the lower first wall 82 as well as the outer peripheral surface of the lower first wall 82. This can increase bonding strength between the reinforcing member 70a and the metal tube 80. Hence, a pressing force applied by the operator in the distal direction can be more efficiently transmitted to the front end of the catheter 10.

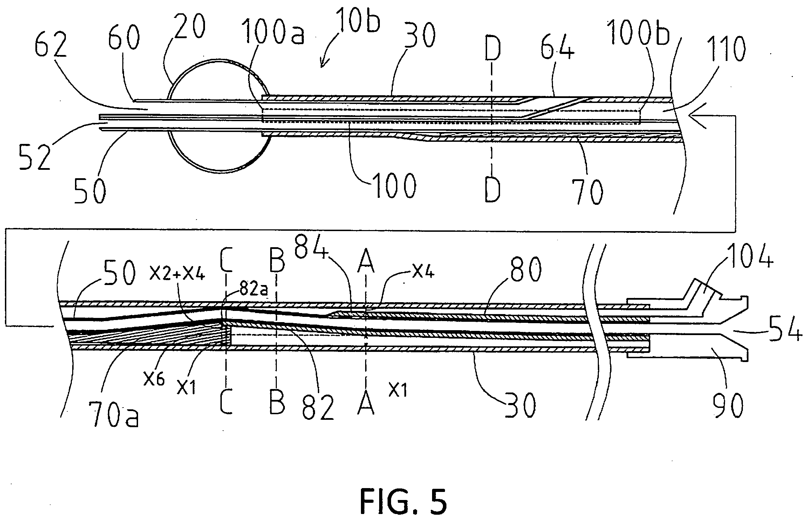

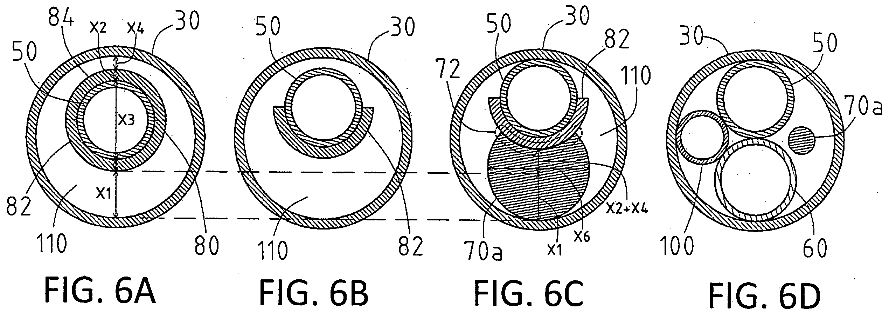

Referring to FIGS. 5 to 6(D), a catheter 10b according to a third embodiment will be described below. As in FIGS. 1 and 3, in FIG. 5, the left side indicates a front end (distal side) to be inserted into a body while the right side indicates a rear end (proximal side) operated by an operator, e.g., a doctor.

Only differences from the catheter 10a in FIGS. 3 to 4 (D) will be described below. In the catheter 10b, a balloon 20 is attached to the front end of an outer tube 30 and the front end of a first inner tube 50. The outer tube 30 has a liquid supply tube 100 for supplying a liquid such as a contrast medium and a physiological saline that inflate the balloon 20, the liquid supply tube 100 being provided along the first inner tube 50 and the second inner tube 60. The liquid supply tube 100 has a front end 100a that is extended into the balloon 20. The liquid supply tube 100 has a rear end 100b that is extended longer than a second insertion opening 64 of the second inner tube 60 toward the rear end of the catheter 10b and is caused to communicate with a lumen 110 formed between the outer tube 30 and the first inner tube 50 (See FIG. 5).

As shown in FIG. 5, when a liquid for inflating the balloon 20 is supplied from an indeflator (not shown) attached to a liquid inlet port 104 of a connector 90, the liquid is supplied to the balloon 20 through the lumen 110 (See FIGS. 6 (A) to 6(C)) on the rear end of the catheter 10b and through the liquid supply tube 100 (See FIG. 6(D)) from a midpoint to the front end of the catheter 10b. The balloon 20 inflated to a blood vessel wall or an alimentary canal wall fixes the front end of the catheter 10b, thereby fixing the front end of the first inner tube 50. This can improve the operability of another medical device inserted into the first inner tube 50.

If a stenosis is located on the end of a blood vessel or an alimentary canal, the front end of the catheter 10b inserted into the blood vessel or the alimentary canal is greatly curved. Thus, the front end of the first inner tube 50 inserted into the outer tube 30 is also greatly curved. In this state, if an operator inserts another medical device into the first inner tube 50, the medical device may break the greatly curved first inner tube 50.

The catheter 10b has the liquid supply tube 100 from a midpoint to the front end of the catheter 10b. Thus, even if an operator inserts another medical device into the first inner tube 50 and erroneously causes the medical device to break the first inner tube 50 with the greatly curved front end, a liquid can supplied to the balloon 20. The liquid supply tube 100 is not provided along the length of the catheter 10b. The rear end 100b of the liquid supply tube 100 is connected to the lumen 110 instead. The lumen 110 is larger in cross-sectional area than the liquid supply tube 100 (See FIGS. 6 (A) to 6(D)). Thus, a liquid passage is extended on the rear end where another medical device is unlikely to break the tube. This configuration can shorten a time period for supplying a liquid into the balloon 20 or a time period for collecting the liquid from the balloon 20, achieving a smooth operation.

In FIGS. 1 to 6(D), the catheters 10, 10a, and 10b each have the second inner tube 60. In the present invention, however, the second inner tube 60 is not always necessary and thus may be optionally omitted.

In the catheters 10, 10a, and 10b, the upper second wall 84 of the metal tube 80 is not provided, the lower first wall 82 is bent to the upper second wall 84, and the rear ends 70 and 70a of the reinforcing members are each joined to the outer peripheral surface of the curved lower first wall 82. Thus, the reinforcing members 70 and 70a increased in diameter at least by the thickness X2 of the upper second wall 84 can be disposed in the catheter 10 without increasing the diameter of the outer tube 30. Hence, when an operator presses the catheter 10 in the distal direction, the catheter 10 is unlikely to have insufficient stiffness in the longitudinal direction so as to reduce the occurrence of breaks on the catheter 10. This can efficiently transmit a pressing force, which is applied by the operator in the distal direction, to the front end of the catheter 10.

### [Reference Signs List]

- 10, 10a, 10b: catheter
- 20: balloon
- 30: outer tube
- 50: first inner tube
- 52: first front-end opening
- 54: first insertion opening
- 60: second inner tube
- 62: second front-end opening
- 64: second insertion opening
- 70, 70a: reinforcing member
- 72: recess
- 80: metal tube
- 82: lower first wall
- 84: upper second wall
- 90: connector
- 100: liquid supply tube
- 104: liquid inlet port
- 110: lumen

## Claims

1. A catheter (10, 10a, 10b) comprising:
an outer tube (30);
a metal tube (80) inserted into the outer tube;
an inner tube (50) inserted into the metal tube; and
a metallic reinforcing member (70, 70a) extended in a longitudinal direction between the outer tube and the inner tube,
wherein:
the metal tube has a lower first wall (82) and an upper second wall (84) opposed to the lower first wall, a front end of the lower first wall is distally extended longer than that of the upper second wall and is bent toward the upper second wall, and
the reinforcing member has a rear end that is joined to an outer peripheral surface of the bent lower first wall.

2. The catheter (10a, 10b) according to claim 1, wherein:
the rear end of the reinforcing member has a recess (72), and
the front end (82a) of the lower first wall is joined to the reinforcing member at the recess.

3. The catheter -(10b) according to claim 1 or 2, further comprising:
a balloon (20) attached to a front end of the inner tube; and
a liquid supply tube (100) provided along the inner tube so as to supply a liquid into the balloon,
wherein:
the liquid supply tube has a rear end (100b) that is connected to a lumen (110) formed between the outer tube and the inner tube.

## Patentansprüche

1. Katheter (10, 10a, 10b) mit:
einem Außenrohr (30);
einem in das Außenrohr eingefügten Metallrohr (80);
einem in das Metallrohr eingefügten Innenrohr (50); und
einem sich in Längsrichtung zwischen dem Außenrohr und dem Innenrohr erstreckenden metallischen Verstärkungsteil (70, 70a),
wobei:
das Metallrohr eine untere erste Wand (82) und eine der unteren ersten Wand gegenüberliegende obere zweiten Wand (84) hat, wobei das vordere Ende der unteren ersten Wand distal länger ist als das der oberen zweiten Wand und in Richtung der oberen zweiten Wand gebogen ist und
das Verstärkungsteil ein hinteres Ende hat, das mit der Außenumfangsfläche der gebogenen unteren ersten Wand verbunden ist.

2. Katheter (10a, 10b) nach Anspruch 1, wobei:
das hintere Ende des Verstärkungsteils eine Aussparung (72) hat, und
das vordere Ende (82a) der unteren ersten Wand in der Aussparung mit dem Verstärkungsteil verbunden ist.

3. Katheter (10b) nach Anspruch 1 oder 2, zusätzlich mit:
einem an das vordere Ende des Innenrohrs angebrachten Ballon (20); und
einem entlang des Innenrohrs vorgesehenen Flüssigkeitszufuhrrohr (100) zur Zufuhr von Flüssigkeit in den Ballon,
wobei:
das Flüssigkeitszufuhrrohr ein hinteres Ende (100b) hat, das mit einem Lumen (110) verbunden ist, das zwischen dem Außenrohr und dem Innenrohr ausgebildet ist.

## Revendications

1. Cathéter (10, 10a, 10b) comprenant :
un tube externe (30) ;
un tube métallique (80) inséré dans le tube externe ;
un tube interne (50) inséré dans le tube métallique ; et
un élément de renforcement métallique (70, 70a) étendu dans une direction longitudinale entre le tube externe et le tube interne,
dans lequel :
le tube métallique possède une première paroi inférieure (82) et une seconde paroi supérieure (84) opposée à la première paroi inférieure, une extrémité avant de la première paroi inférieure est plus étendue distalement que celle de la seconde paroi supérieure et est courbée vers la seconde paroi supérieure, et
l'élément de renforcement possède une extrémité arrière qui est jointe à une surface périphérique externe de la première paroi inférieure courbée.

2. Cathéter (10a, 10b) selon la revendication 1, dans lequel :
l'extrémité arrière de l'élément de renforcement présente un évidement (72), et
l'extrémité avant (82a) de la première paroi inférieure est jointe à l'élément de renforcement au niveau de l'évidement.

3. Cathéter (10b) selon la revendication 1 ou 2, comprenant en outre :
un ballonnet (20) fixé à une extrémité avant du tube interne ; et
un tube d'alimentation en liquide (100) disposé le long du tube interne de sorte à fournir un liquide au ballonnet,
dans lequel :
le tube d'alimentation en liquide possède une extrémité arrière (100b) qui est reliée à une lumière (110) formée entre le tube externe et le tube interne.
